# FASCICULE DE BREVET EUROPEEN

(11) **EP 3 820 450 B1**
(45) Date de publication et mention de la délivrance du brevet: **21.08.2024**
(21) Numéro de dépôt: 19798314.1
(22) Date de dépôt: 08.11.2019
(51) Int. Cl.: A61K 9/08, A61K 9/00, A61K 47/02, A61K 47/26, A61K 31/047, A61P 11/02

(54) **COMPOSITION AQUEUSE SALINE, SON PROCEDE DE FABRICATION, ET SON UTILISATION**
WÄSSRIGE SALZHALTIGE ZUSAMMENSETZUNG, DEREN HERSTELLUHNGSPROZESS UND DEREN VERWENDUNG
AQUEOUS SALINE COMPOSITION, ITS PROCESS OF MANUFACTURING AND ITS USE

(30) Priorité: 13.11.2018 FR 1871509
(43) Date de publication de la demande: 19.05.2021
(73) Titulaire: Djellouli, Saïd, 75008 Paris (FR); Fox, Andrea, 75008 Paris (FR)
(72) Inventeur: Djellouli, Saïd, 75008 Paris (FR); Fox, Andrea, 75008 Paris (FR)
(74) Mandataire: Fidal Innovation
(86) Numéro de dépôt international: PCT/EP2019/080694
(87) Numéro de publication internationale: WO 2020/099264

(56) Documents cités:
- EP-A1- 3 369 429
- WO-A1-2008/037938
- WO-A1-98/08500

## Description

L'objet de la présente invention concerne une composition aqueuse saline, son procédé d'obtention, son utilisation comme médicament par exemple sous forme de pulvérisateur pour la prévention ou le traitement d'appoint des affections oto-rhino-laryngologiques (ORL).

Les fosses nasales agissent comme un filtre qui permet de protéger contre les affections oto-rhino-laryngologiques, telles que sinusites, rhinites, etc. Or, différentes compositions à base d'eau salée, telle que l'eau de mer, sont connues pour le lavage de fosses nasales, en particulier dans le cadre de telles pathologies.

Par exemple, de telles compositions à base d'eau de mer sont généralement prévues pour être isotoniques. Ces compositions peuvent être obtenues par le mélange d'eau de mer avec de l'eau purifiée, ou par extraction de certains ions (sodium, potassium, chlorure, etc. - par exemple par électrodialyse). Ces mélanges peuvent être soumis à des procédures de stérilisation, telles qu'une filtration (préférentiellement), soumission à rayonnement UV, avant leur conditionnement. Les compositions finalement obtenues peuvent être également radio-stérilisés (rayonnement beta ou gamma). Les produits sont conditionnés sous la forme de pulvérisateurs aérosols ou de pulvérisateurs mécaniques. Le document FR 2 299 041 concerne une telle composition. Le document WO 2008/037938 décrit une solution aqueuse saline hypertonique à base uniquement d'eau de mer, et ayant un rapport Na⁺/Mg²⁺ inférieur à 6.

Dans un domaine thérapeutique totalement distinct, le document EP 3 369 429 décrit une composition saline comprenant une ocytocine et un polymère carboxyvinile, et une osmolalité inférieure à celle du sérum physiologique, c'est-à-dire inférieure à 286 mosmol/kg.

Dans un domaine thérapeutique totalement distinct, le document WO 98/08500 décrit une composition aqueuse saline contenant de l'arginine-L, pour corriger les ischémies cérébrales et les hypoxies dans le cas de combinaisons de trauma au cerveau (TBI) et d'hémorragies.

La présente invention vise à améliorer l'efficacité des compositions connues à base d'eau de mer.

### RESUME DE L'INVENTION

L'objet de la présente invention concerne une composition aqueuse saline comprenant un composé organique filmo-protecteur et/ou hyper-osmotique pour son utilisation dans le traitement et la prévention des affections oto-rhino-laryngologiques, dans laquelle l'osmolarité de ladite composition est supérieure à 1000 mosmol/l à 20°C et inférieure à 2000 mosmol/l à 20°C, dans laquelle le composé organique filmo-protecteur et/ou hyper-osmotique est le xylitol.

Selon un exemple de réalisation, l'osmolarité de ladite composition est supérieure à 1500 mosmol/l à 20°C.

L'objet de la présente invention concerne également un procédé de fabrication d'une composition aqueuse comprenant un composé organique filmo-protecteur et/ou hyper-osmotique comprenant un polyol dérivé d'un composé saccharide, le composé saccharide en question, ou un mélange de ceux-ci, et d'osmolarité supérieure à 1000 mosmol/l à 20°C, comprenant les étapes suivantes :
a) micro-filtration d'eau de mer ;
b) ajout d'eau purifiée,
c) mélange
d) tout en mélangeant, ajout dans la solution de l'étape c) du composé organique filmo-protecteur et/ou hyper-osmotique;
e) récupération du mélange de l'étape d) ;
   ledit procédé étant caractérisé en ce que la quantité d'eau de mer est supérieure ou égale à 10% en masse totale de composition aqueuse obtenue à l'étape e) et en ce que la quantité de composé organique filmo-protecteur et/ou hyper-osmotique est supérieure ou égale à 5% en masse totale de composition aqueuse obtenue à l'étape e), et dans lequel le composé organique filmo-protecteur et/ou hyper-osmotique est le xylitol.

Selon un aspect, la quantité d'eau de mer est supérieure ou égale à 15%, 20%, 30%, 40%, 50%, 60%, 70% ou 80% en masse totale de composition aqueuse obtenue à l'étape e), et/ou la quantité de composé organique filmo-protecteur et/ou hyper-osmotique est supérieure ou égale à 6%, 7%, 8%, 9%, 10%, 15%, 20%, préférentiellement de 11%, en masse totale de composition aqueuse obtenue à l'étape e).

Ainsi, l'objet de la présente invention concerne une composition susceptible d'être obtenue par le procédé de fabrication décrit ci-dessus.

Selon un aspect, la composition comprend :
- une concentration en chlorures comprise entre 2 et 18 g/kg
- une concentration en sodium comprise entre 1 et 10 g/kg
- une concentration en sulfates comprise entre 0,3 et 2,4 g/kg
- une concentration en magnésium comprise entre 0,1 et 1,2 g/kg
- une concentration en calcium comprise entre 0,04 et 0,4 g/kg
- une concentration en potassium comprise entre 0,04 et 0,4 g/kg
- et/ou elle a un pH compris entre 4 et 10 de préférence entre 5 et 9, par exemple 7.

Selon un aspect, la composition se présente sous des formes posologiques finales choisies dans le groupe constitué de gouttes nasales, de pulvérisations nasales liquides et de lavages nasaux.

De plus, l'objet de la présente invention concerne une composition telle que décrite présentement en tant que médicament présentant un effet thérapeutique. Il concerne une telle composition pour son utilisation dans le traitement des affections des muqueuses nasales d'origine virale, bactérienne ou allergiques.

Selon un aspect, la composition comprend de l'eau de mer.

Plus précisément, l'objet de la présente invention concerne une composition telle que décrite présentement, destinée à son utilisation dans le traitement et la prévention des affections oto-rhino-laryngologiques, notamment pour la prévention de l'otite moyenne.

Enfin, l'objet de la présente invention concerne un dispositif d'administration nasal, tel qu'un pulvérisateur, caractérisé en ce qu'il comprend une composition selon la présente invention.

### DEFINITIONS

Par « composé organique filmo-protecteur », il est compris dans le cadre de la présente invention un composé ayant la propriété de protéger la surface biologique à laquelle ledit composé est appliqué. Dans le cas d'espèce, ce composé protège l'épithélium des fosses nasales et améliore le fonctionnement des cils vibratiles. Ce composé comprend au moins une entité « C-H », où « C » représente un atome de carbone et « H » représente un atome d'hydrogène. Ce composé peut notamment être un polyol. Il s'agit d'un dérivé d'un composé saccharide. En particulier, le composé peut être mono-saccharide. Le monosaccharide peut être un pentose. Selon certains exemples, le composé organique filmo-protecteur est le xylitol. Ce composé organique filmo-protecteur peut également, ou alternativement, être associé ou remplacé par le saccharide correspondant. Notamment, en ce qui concerne le xylitol, il pourrait être associé à ou remplacé par le xylose. Le composé organique filmo-protecteur peut être obtenu par voie de synthèse, ou par extraction d'un produit naturel.

Par « tonicité » d'une solution, dans le cadre de l'invention, on fait référence à la concentration en sel dans celle-ci par rapport au sang humain. Ainsi, par « isotonique », on désigne une solution dont la concentration en soluté est égale à celle contenue dans le sang. Par « hypotonique », on désigne une solution dont la concentration en soluté est inférieure à celle contenue dans le sang. Par « hypertonique », on désigne une solution dont la concentration en soluté est supérieure à celle contenue dans le sang.

Par « composé hyper-osmotique », il est compris dans le cadre de la présente invention un composé possédant un pouvoir osmotique élevé ou qui résulte d'une osmose très rapide, ou qui la provoque. L'osmose est un phénomène de diffusion de la matière, mis en évidence lorsque des molécules de solvant traversent une membrane semi-perméable séparant deux solutions dont les concentrations en soluté sont différentes. Le transfert global de solvant se fait alors de la solution la moins concentrée vers la solution la plus concentrée jusqu'à l'équilibre. Ce composé est organique, c'est-à-dire qu'il comprend au moins une entité « C-H », où « C » représente un atome de carbone et « H » représente un atome d'hydrogène. Ce composé est notamment un polyol. Il s'agit d'un composé dérivé d'un composé saccharide. Le composé saccharide est mono-saccharide.Le monosaccharide est un pentose. Le composé estle xylitol. Ce composé organique filmo-protecteur peut également, ou alternativement, être associé ou remplacé par le saccharide correspondant. Notamment, en ce qui concerne le xylitol, il pourrait être associé à ou remplacé par le xylose. Le composé organique hyper-osmotique peut être obtenu par voie de synthèse, ou par extraction d'un produit naturel. Afin de déterminer si un composé est hyper-osmotique selon la présente invention, un test peut être réalisé au moyen d'un osmomètre. On mesure par exemple l'osmolarité à l'aide d'un osmomètre cryoscopique, par mesure du point de congélation, par référence avec celui de l'eau distillée. D'autres technologies d'osmométrie sont connues, qui peuvent être calibrées pour obtenir des résultats similaires aux osmomètres cryoscopiques.

Par « composition aqueuse saline », il est compris dans le cadre de la présente invention une composition comprenant de l'eau et du sel. Si la composition aqueuse saline est préférentiellement une composition à base d'eau de mer, tout type de sel ayant les mêmes propriétés que les sels trouvés dans l'eau de mer, une fois dissous dans de l'eau, peuvent être utilisées. Préférentiellement il est compris par « composition aqueuse saline » une composition comprenant de l'eau et au moins un sel trouvé dans l'eau de mer. Préférentiellement, le sel est NaCl. L'eau de mer peut également comprendre d'autres éléments, comme par exemple le sulfate, le magnésium, le calcium, le potassium, le cuivre, le fer et le zinc.

Par « osmolalité », il est compris dans le cadre de la présente invention, le nombre d'osmoles de soluté par kilogramme de solvant. Par « osmolarité », il est compris dans le cadre de la présente invention, le nombre d'osmoles de soluté par litre de solution. Une osmole est égale au nombre de moles de particules qui peuvent être osmotiquement actives dans une solution idéale.

### DESCRIPTION DETAILLEE

Procédé de fabrication d'une composition aqueuse selon l'invention

L'objet de la présente invention concerne donc un procédé de fabrication d'une composition aqueuse telle que décrit ci-dessus.

Selon un mode de réalisation, le procédé de fabrication comprend les étapes suivantes :
a) micro-filtration d'eau de mer ;
b) ajout d'eau purifiée ;
c) mélange
d) tout en mélangeant, ajout dans la solution de l'étape b) du composé organique filmo-protecteur et/ou hyper-osmotique;
e) récupération du mélange de l'étape d).

De manière préférée, le procédé de fabrication d'une composition aqueuse selon la présente invention peut être caractérisée en ce que la quantité d'eau de mer est supérieure ou égale à 10%, 15%, 20%, 30%, 40%, 50%, 60%, 70% ou 80% en masse totale de composition aqueuse obtenue à l'étape e), et/ou que la quantité de composé organique filmo-protecteur et/ou hyper-osmotique est supérieure ou égale à 5%, 6%, 7%, 8%, 9%, 10%, 15%, 20%, préférentiellement de 11%, en masse totale de composition aqueuse obtenue à l'étape e).

Le procédé de fabrication d'une composition aqueuse selon la présente invention est caractérisé en ce que le composé organique filmo-protecteur et/ou hyper-osmotique est un polyol, notamment dérivé d'un saccharide.

Le procédé de fabrication d'une composition aqueuse selon la présente invention est caractérisé en ce que le composé organique filmo-protecteur et/ou hyper-osmotique est un monosaccharide et/ou un polyol dérivé de ce monosaccharide, ou un mélange de ceux-ci.

Le procédé de fabrication d'une composition aqueuse selon la présente invention est caractérisé en ce que le composé organique filmo-protecteur et/ou hyper-osmotique est le xylitol, voire un mélange de xylitol avec un composé choisi parmi le maltitol, le mannitol, le sorbitol, le lactitol, le galactitol, l'erythritol, l'arabitol et le ribitol. Ce(s) composé(s) organique(s) filmo-protecteur(s) peu(ven)t également, ou alternativement, être associé(s) ou remplacé(s) par le(s) saccharide(s) correspondant. Notamment, en ce qui concerne le xylitol, il pourrait être associé à ou remplacé par le xylose.

Dans un mode de réalisation particulier, le procédé de fabrication d'une composition aqueuse selon la présente invention peut être caractérisée en ce que l'étape a) est effectuée par une filtration d'eau de mer à travers un filtre de 0,2 µm, voire à travers un filtre stérilisant de 0,1 µm.

### Composition

L'objet de la présente invention concerne une composition aqueuse saline telle que décrite ci-dessus, c'est-à-dire caractérisable par exemple par son osmolarité. Un mode de réalisation particulier concerne une composition aqueuse saline susceptible d'être obtenue par le procédé décrit ci-dessus. En effet, l'un des modes de réalisation selon la présente invention concerne l'utilisation d'eau de mer, pouvant comprendre une variété d'espèces chimiques potentiellement actives.

Ainsi, dans un mode de réalisation particulier, la composition aqueuse selon la présente invention peut être caractérisée en ce que ladite composition comprend :
- une concentration en chlorures comprise entre 2 et 18 g/kg
- une concentration en sodium comprise entre 1 et 10 g/kg
- une concentration en sulfates comprise entre 0,3 et 2,4 g/kg
- une concentration en magnésium comprise entre 0,1 et 1,2 g/kg
- une concentration en calcium comprise entre 0,04 et 0,4 g/kg
- une concentration en potassium comprise entre 0,04 et 0,4 g/kg
et/ou qu'elle a un pH compris entre 4 et 10 de préférence entre 5 et 9, par exemple 7.

La composition aqueuse peut également comprendre du cuivre. La composition aqueuse peut également comprendre du zinc.

De manière préférée, la composition aqueuse selon la présente invention peut être caractérisée en ce que ladite composition comprend de l'eau de mer.

De manière préférée, la composition aqueuse selon la présente invention peut être caractérisée en ce qu'elle peut former des formes posologiques finales choisies dans le groupe constitué de gouttes nasales, de pulvérisations nasales, et de lavages nasaux.

Dans un mode de réalisation particulier, la composition aqueuse selon la présente invention peut être caractérisée en ce que le composé organique filmo-protecteur et/ou hyper-osmotique est le xylitol. Ce composé organique filmo-protecteur peut également, ou alternativement, être associé ou remplacé par le saccharide correspondant. Notamment, en ce qui concerne le xylitol, il pourrait être associé à ou remplacé par le xylose.

De manière préférée, la composition aqueuse selon la présente invention peut être caractérisée en ce que le composé organique filmo-protecteur et/ou hyper-osmotique est compris à une concentration supérieure ou égale à 5% en masse, préférentiellement comprise entre 10 et 20% en masse par rapport à la masse totale de composition, notamment une composition de 11% en masse.

Selon un exemple de réalisation, aucun conservateur chimique n'a été ajouté dans la composition chimique.

L'ajout d'un ou de plusieurs excipients est tout à fait possible, comme par exemple des extraits aqueux de plantes cosmétiques ou aromatiques.

### Application thérapeutique

La composition selon la présente invention peut donc être utilisée comme médicament. La composition selon la présente invention peut être destinée à son utilisation pour la prévention ou le traitement d'appoint des affections oto-rhino-laryngologiques (ORL). La composition peut également être destinée à la prévention des otites moyennes.

Plus précisément, la composition selon la présente invention peut être destinée à son utilisation dans le traitement des affections des muqueuses nasales d'origine virale, bactérienne ou allergique, telles que les sinusites, les rhinites, les polypes nasaux, les fibroses kystiques, etc...

La composition selon la présente invention peut également être destinée à son utilisation dans la prévention de l'otite moyenne. De manière préférée, la composition aqueuse selon la présente invention peut être caractérisée en ce que l'affection bactérienne est due à au moins l'un des pathogènes choisi dans le groupe consistant principalement en Streptococcus pneumoniae, Haemophilus influenzae, Moraxella catarrhalis, Staphylococcus aureus.

Sans vouloir être limités par la théorie, les inventeurs ont constaté de manière surprenante que la composition objet de l'invention présente une synergie entre les différents effets bénéfiques allant au-delà des effets individuels apportés par chacun des éléments, à savoir :
. un nettoyage mécanique en profondeur par l'eau de mer, qui va entraîner avec elle les particules virales et allergènes, protéger l'épithélium des fosses nasales, et améliorer le fonctionnement des cils vibratiles,
. un effet protecteur apporté par certains composants de l'eau de mer, comme décrit plus en détail ci-dessous,
. et un effet de barrière contre la progression des bactéries vers la trompe d'Eustache apporté par le composé filmo-protecteur.

Les oligo-éléments contenus dans l'eau de mer (comme le zinc, le cuivre, ou le magnésium) contribuent à une meilleure défense de l'organisme face aux agressions bactériennes et virales, et permettent de réduire le risque des infections respiratoires basses. Six études ont été menées pour examiner les effets de la supplémentation en zinc dans la prévention de la pneumonie, au Bangladesh, en Inde, au Pérou et en Afrique du Sud. 5193 enfants âgés de 2 à 59 mois ont été suivis. La supplémentation en zinc est associée de façon significative à une réduction de l'incidence et de la prévalence de la pneumonie chez les enfants âgés de 2 à 59 mois.

Le xylitol/xylose du composé filmo-protecteur fournit un effet de barrière contre la progression des bactéries vers la trompe d'Eustache, et assure ainsi une protection contre l'otite moyenne. Le maltitol, le mannitol, le sorbitol, le lactitol, le galactitol, l'erythritol, l'arabitol, ou le ribitol peuvent avoir des effets similaires au xylitol/xylose.

### Dispositif d'administration

L'objet de la présente invention concerne enfin un dispositif d'administration nasal, tel qu'un pulvérisateur, caractérisé en ce qu'il comprend une composition selon la présente invention.

Tout type de dispositif d'administration nasal peut être utilisé. On peut par exemple envisager une pompe mécanique ou un pulvérisateur aérosol, avec ou sans valve à poche. De façon préférentielle, on utilise un pulvérisateur aérosol avec une valve à poche.

De manière avantageuse, la poche est sous pression, par exemple supérieur ou égal à 1,5 bars, 2 bars, 3 bars, 4 bars, 5 bars ou encore 10 bars.

Les dispositifs d'administration sous forme de pulvérisateurs aérosol avec valve à poche sont conçus pour limiter et éviter tout risque de rétrocontamination. Cette propriété a été vérifiée par des tests appropriés.

### EXEMPLES

Les exemples donnés ci-dessous sont une simple illustration de la présente invention qui n'est pas limitée à ces modes de réalisations particuliers.

Plusieurs compositions ont été réalisées, et leur osmolarité mesurée, comme représenté dans le tableau 1 ci-dessous.

**[Tableaux 1]**

| Compositions | A | B | C | D |
|---|---|---|---|---|
| Xylitol | 11% | 11% | - | |
| Eau de mer | 21% | 68% | 30% | 80% |
| Eau purifiée | 68% | 21% | 70% | 20% |
| Osmolarité moyenne (mosmol/l) | 1100 | 1700 | 300 | 800 |

Les quantités de matière sont données en % m/m à 20°C. L'osmolarité moyenne correspond à la moyenne des osmolalités mesurées pour différents échantillons de chaque composition.

Les compositions A et B correspondent à des modes de réalisation de la présente invention. La composition C est une solution isotonique d'eau de mer et la composition D est une solution hypertonique d'eau de mer.

La solution A, bien qu'hypotonique, présente une osmolarité très supérieure à celle du sang.

La solution B, hypertonique, présente une osmolarité très supérieure à celle du sang (quasiment 6 fois plus).

Des tests ont été menés, pendant lesquels les compositions ont été administrées par pulvérisateur nasal à des sujets de test selon différentes posologies d'application.

La composition C a des effets nettoyants, purifiants, et cicatrisants.

La composition D a un effet de décongestion du nez.

La composition A est efficace pour la protection contre l'otite moyenne des patients. La présence d'eau de mer permet d'élargir l'effet protecteur à l'ensemble de la sphère ORL. L'eau de mer a un effet nettoyant, purificateur, et cicatrisant de la muqueuse nasale.

La composition B associe les effets bénéfiques de la présence d'un polyol (assurant la protection contre les otites moyennes) à une forte concentration d'eau de mer. Les effets synergiques sont démultipliés et inattendus.. L'hyperosmolarité explique les effets décongestionnants extraordinaires constatés lors des essais effectués avec des lots pilotes. En effet, une seule pulvérisation dans chaque narine avec la solution B permet de décongestionner le nez pendant une durée relativement longue, en fonction de l'état physiologique du patient (de une à plusieurs heures). Il existe de nombreuses applications de la solution B. Parmi elles, la solution B permet de remplacer bénéfiquement et de façon naturelle, les produits décongestionnants de formulation purement chimique comme les corticostéroïdes ou les vasoconstricteurs, connus pour leurs effets secondaires à court ou plus long terme (tachycardie, hypertension artérielle, dérèglements hormonaux, addiction). De façon totalement opposée aux produits purement chimiques, la solution B contribue à une amélioration de la fonction respiratoire à court et surtout à long terme, avec une action préventive sur les affections oro-rhino-laryngologiques, et sans effets secondaires. Une pulvérisation dans chaque narine avant le coucher permet de décongestionner durablement le nez et mieux dormir grâce à une respiration plus fluide. Une application dans la réduction du ronflement et des apnées du sommeil est parfaitement envisageable, avec comme corollaire une meilleure hygiène de vie et une baisse globale de la pression artérielle, grâce à un sommeil de meilleure qualité.

## Revendications

1. Composition aqueuse saline comprenant un composé organique filmo-protecteur et/ou hyper-osmotique pour son utilisation dans le traitement et la prévention des affections oto-rhino-laryngologiques, dans laquelle l'osmolarité de ladite composition est supérieure à 1000 mosmol/l à 20°C, notamment supérieure à 1500 mosmol/l à 20°C, et inférieure à 2000 mosmol/l à 20°C, dans laquelle le composé organique filmo-protecteur et/ou hyper-osmotique est le xylitol.

2. Procédé de fabrication d'une composition aqueuse comprenant un composé organique filmo-protecteur et/ou hyper-osmotique et d'osmolarité supérieure à 1000 mosmol/l à 20°C, comprenant les étapes suivantes :
a) micro-filtration d'eau de mer ;
b) ajout d'eau purifiée ;
c) mélange ;
d) tout en mélangeant, ajout dans la solution de l'étape c) du composé organique filmo-protecteur et/ou hyper-osmotique;
e) récupération du mélange de l'étape d) ;
ledit procédé étant **caractérisé en ce que** la quantité d'eau de mer est supérieure ou égale à 10% en masse totale de composition aqueuse obtenue à l'étape e) et **en ce que** la quantité de composé organique filmo-protecteur et/ou hyper-osmotique est supérieure ou égale à 5% en masse totale de composition aqueuse obtenue à l'étape e), et dans lequel le composé organique filmo-protecteur et/ou hyper-osmotique est le xylitol..

3. Procédé de fabrication d'une composition aqueuse selon la revendication 2, **caractérisée en ce que** la quantité d'eau de mer est supérieure ou égale à 15%, 20%, 30%, 40%, 50%, 60%, 70% ou 80% en masse totale de composition aqueuse obtenue à l'étape e), et/ou que la quantité de composé organique filmo-protecteur et/ou hyper-osmotique est supérieure ou égale à 6%, 7%, 8%, 9%, 10%, 15%, 20%, préférentiellement de 11%, en masse totale de composition aqueuse obtenue à l'étape e).

4. Composition susceptible d'être obtenue par l'une quelconque des revendications 2 ou 3.

5. Composition selon la revendication 1 ou 4 **caractérisée en ce que** ladite composition comprend :
- une concentration en chlorures comprise entre 2 et 18 g/kg
- une concentration en sodium comprise entre 1 et 10 g/kg
- une concentration en sulfates comprise entre 0,3 et 2,4 g/kg
- une concentration en magnésium comprise entre 0,1 et 1,2 g/kg
- une concentration en calcium comprise entre 0,04 et 0,4 g/kg
- une concentration en potassium comprise entre 0,04 et 0,4 g/kg
- et/ou qu'elle a un pH compris entre 4 et 10 de préférence entre 5 et 9, par exemple 7.

6. Composition selon la revendication 1, 4 ou 5, **caractérisée en ce qu'**elle se présente sous des formes posologiques finales choisies dans le groupe constitué de gouttes nasales, de pulvérisations nasales liquides et de lavages nasaux.

7. Composition selon l'une quelconque des revendications 1, ou 4 à 6, pour son utilisation dans le traitement des affections des muqueuses nasales d'origine virale, bactérienne ou allergiques.

8. Composition selon l'une quelconque des revendications 1, ou 4 à 6, pour son utilisation dans la prévention de l'otite moyenne.

9. Composition selon l'une quelconque des revendications 1, ou 4 à 8, **caractérisée en ce que** la composition comprend de l'eau de mer.

10. Dispositif d'administration nasal, tel qu'un pulvérisateur, **caractérisé en ce qu'**il comprend une composition selon l'une quelconque des revendications 1, ou 4 à 9.

## Patentansprüche

1. Wässrige salzhaltige Zusammensetzung, die eine filmschützende und/oder hyperosmotische organische Verbindung umfasst, zur Verwendung bei der Behandlung und Vorbeugung von Hals-Nasen-Ohren-Erkrankungen, wobei die Osmolarität der genannten Zusammensetzung über 1000 mosmol/l bei 20 °C, insbesondere über 1500 mosmol/l bei 20 °C und unter 2000 mosmol/l bei 20 °C liegt, wobei die filmschützende und/oder hyperosmotische organische Verbindung Xylit ist.

2. Verfahren zur Herstellung einer wässrigen Zusammensetzung, die eine filmschützende und/oder hyperosmotische organische Verbindung umfasst und eine Osmolarität von mehr als 1000 mosmol/l bei 20 °C aufweist, das die folgenden Schritte beinhaltet:
a) Mikrofiltrieren von Meerwasser;
b) Zugeben von gereinigtem Wasser;
c) Mischen;
d) Zugeben, unter Mischen, der filmschützenden und/oder hyperosmotischen organischen Verbindung zu der Lösung aus Schritt c);
e) Gewinnen der Mischung aus Schritt d);
wobei das Verfahren **dadurch gekennzeichnet ist, dass** die Menge an Meerwasser gleich oder größer 10 % der Gesamtmasse der in Schritt e) erhaltenen wässrigen Zusammensetzung ist, und dadurch, dass die Menge an filmschützender und/oder hyperosmotischer organischer Verbindung gleich oder größer 5 % der Gesamtmasse der in Schritt e) erhaltenen wässrigen Zusammensetzung ist, und wobei die filmschützende und/oder hyperosmotische organische Verbindung Xylit ist.

3. Verfahren zur Herstellung einer wässrigen Zusammensetzung nach Anspruch 2, **dadurch gekennzeichnet, dass** die Menge an Meerwasser gleich oder größer 15 %, 20 %, 30 %, 40 %, 50 %, 60 %, 70 % oder 80 % der Gesamtmasse der in Schritt e) erhaltenen wässrigen Zusammensetzung ist, und/oder dass die Menge an filmschützender und/oder hyperosmotischer organischer Verbindung gleich oder größer 6 %, 7 %, 8 %, 9 %, 10 %, 15 %, 20 %, vorzugsweise 11 %, der Gesamtmasse der in Schritt e) erhaltenen wässrigen Zusammensetzung ist.

4. Zusammensetzung, die im Stande ist, gemäß einem der Ansprüche 2 oder 3 erhalten zu werden.

5. Zusammensetzung nach Anspruch 1 oder 4, **dadurch gekennzeichnet, dass** die genannte Zusammensetzung Folgendes umfasst:
- eine Chloridkonzentration zwischen 2 und 18 g/kg
- eine Natriumkonzentration zwischen 1 und 10 g/kg
- eine Sulfatkonzentration zwischen 0,3 und 2,4 g/kg
- eine Magnesiumkonzentration zwischen 0,1 und 1,2 g/kg
- eine Kalziumkonzentration zwischen 0,04 und 0,4 g/kg
- eine Kaliumkonzentration zwischen 0,04 und 0,4 g/kg
- und/oder dadurch, dass sie einen pH-Wert zwischen 4 und 10, vorzugsweise zwischen 5 und 9, beispielsweise 7, aufweist.

6. Zusammensetzung nach Anspruch 1, 4 oder 5, **dadurch gekennzeichnet, dass** sie in endgültigen Dosierungsformen ausgewählt aus der Gruppe bestehend aus Nasentropfen, flüssigen Nasensprays und Nasenspülungen vorliegt.

7. Zusammensetzung nach einem der Ansprüche 1 oder 4 bis 6 zur Verwendung bei der Behandlung von viral, bakteriell oder allergisch bedingten Erkrankungen der Nasenschleimhäute.

8. Zusammensetzung nach einem der Ansprüche 1 oder 4 bis 6 zur Verwendung bei der Vorbeugung von Mittelohrentzündung.

9. Zusammensetzung nach einem der Ansprüche 1 oder 4 bis 8, **dadurch gekennzeichnet, dass** die Zusammensetzung Meerwasser umfasst.

10. Nasale Verabreichungsvorrichtung, wie z.B. ein Zerstäuber, **dadurch gekennzeichnet, dass** sie eine Zusammensetzung nach einem der Ansprüche 1 oder 4 bis 9 enthält.

## Claims

1. The aqueous saline composition comprising an organic film-protective and/or hyperosmotic, for its use in the treatment and prevention of ear, nose and throat (ENT) conditions wherein the osmolarity of said composition is higher than 1,000 mosmol/l at 20°C, particularly higher than 1,500 mosmol/l at 20°C and, less than 2,000 mosmol/l at 20°C, wherein the organic film-protective and/or hyperosmotic compound is xylitol.

2. The manufacturing process of an aqueous composition comprising an organic film-protective and/or hyperosmotic compound and having an osmolarity greater than 1,000 mosmol/l at 20°C, comprising the following steps:
a) microfiltration of seawater;
b) addition of purified water;
c) mixing;
d) while mixing, addition to the solution from step c) of the organic film-protective and/or hyperosmotic compound ;
e) recovery of the mixture from step d) ;
said process being **characterised in that** the quantity of seawater is higher than or equal to 10% of the total mass of the aqueous composition obtained in step e) and **in that** the quantity of organic film-protective and/or hyperosmotic compound is higher than or equal to 5% of the total mass of the aqueous composition obtained in step e), and wherein the organic film-protective and/or hyperosmotic compound is xylitol.

3. The manufacturing process of an aqueous composition according to claim 2, **characterised in that** the quantity of seawater is higher than or equal to 15%, 20%, 30%, 40%, 50%, 60%, 70% or 80% of the total mass of the aqueous composition obtained in step e) and/or the quantity of organic film-protective and/or hyperosmotic compound is higher than or equal to 6%, 7%, 8%, 9%, 10%, 15%, 20%, preferentially 11%, of the total mass of the aqueous composition obtained in step e).

4. Composition obtainable by any one of claims 2 or 3.

5. Composition according to claims 1 or 4 **characterised in that** said composition comprises:
- a chloride concentration between 2 and 18 g/kg
- a sodium concentration between 1 and 10 g/kg
- a sulphate concentration between 0.3 and 2.4 g/kg
- a magnesium concentration between 0.1 and 1.2 g/kg
- a calcium concentration between 0.04 and 0.4 g/kg
- a potassium concentration between 0.04 and 0.4 g/kg
- and/or a pH between 4 and 10 preferably between 5 and 9, for example 7.

6. Composition according to claims 1, 4 or 5, **characterised in that** it is in final dosage forms selected from the group consisting of nasal drops, liquid nasal sprays and nasal washes.

7. Composition according to any one of claims 1, or 4 to 6, for its use in the treatment of nasal mucosal conditions of viral, bacterial or allergic origin.

8. Composition according to any one of claims 1, or 4 to 6, for its use in the prevention of otitis media.

9. Composition according to any one of claims 1, or 4 to 8, **characterised in that** the composition comprises seawater.

10. Nasal administration device, such as a spray device, **characterised in that** it comprises a composition according to any one of claims 1, or 4 to 9.
